# DEMANDE DE BREVET EUROPEEN

(11) **EP 0 947 173 A1**
(43) Date de publication de la demande: **06.10.1999**
(21) Numéro de dépôt: 98810275.2
(22) Date de dépôt: 30.03.1998
(51) Int. Cl.: A61B 17/41

(54) **Sonde pour le traitement haute fréquence de la peau**

(71) Demandeur: Bernaz, Gabriel, 1232 Confignon (CH)
(72) Inventeur: Bernaz, Gabriel, 1232 Confignon (CH)
(74) Mandataire: Munzinger, John Patrick

(57) **Abrégé**

Une sonde pour l'application d'un flux d'énergie électromagnétique haute fréquence sur la peau à travers un mélange de gel conducteur et de produit traitant, destinée à faire pénétrer le produit traitant dans la peau. La sonde est pourvue d'une surface d'appui non-conductrice (14), présentant une pluralité de cavités (6) autour ou au voisinage desquels des parties d'éléments inducteurs (5) disposés en couches successives émettent un champ électromagnétique. Les cavités (6) débouchent par un orifice (3) et contiennent le mélange gel/produit traitant dont la pénétration cutanée est activée par le champ électromagnétique.

## Description

### DOMAINE TECHNIQUE

L'invention se rapporte au traitement de la peau par des moyens électriques à haute fréquence associés ou non au rayonnement électromagnétique laser, en particulier pour l'épilation dite "définitive" ou "longue durée", ainsi que pour la repousse des cheveux.

### ETAT DE LA TECHNIQUE

WO 93/04636 décrit un procédé basé sur la constatation qu'en mélangeant un gel conducteur de type usuel pour l'application de sondes à ultrasons contre la peau avec un produit traitant, par exemple une lotion à effet d'atrophie sur les racines des poils, il était possible, par induction transcutanée à haute fréquence, de faire pénétrer le produit traitant dans les follicules pileux (pores)et la tige des poils, et ainsi d'effectuer un traitement.

Ce procédé permet ainsi d'effectuer un traitement, notamment cosmétique, de la peau, par exemple en vue d'une épilation progressivement définitive voire longue durée, et permet de surcroît une application ponctuelle et efficace au niveau des follicules, sans intervention manuelle délicate.

Pour la mise en oeuvre du procédé, WO 93/04636 décrit en outre un appareil comprenant un organe maniable de contact avec la peau ayant un corps non-conducteur pourvu d'une surface d'appui destinée à être appliquée sur la peau. Cette surface comporte une pluralité de points conducteurs d'émission électromagnétique distincts formés par exemple par des parties exposées de spires d'un solénoïde noyé dans le corps de l'organe de contact. Ces points débouchent par des orifices dans cette surface, de préférence en retrait par rapport à celle-ci, de manière que, en cours d'utilisation de l'appareil, ils puissent contacter du gel conducteur appliqué sur la peau. Ces points d'émission distincts émettent un flux d'énergie électromagnétique à haute fréquence, avantageusement un courant émetteur pur, fourni par un circuit électrique oscillant haute fréquence de puissance.

Pour la mise en oeuvre du même procédé, WO 96/03928 décrit une sonde flexible susceptible de se conformer à la partie du corps contre laquelle elle est appliquée, comportant des parties non-conductrices inférieure/ interne et supérieure/ externe. Au moins la partie inférieure/ interne est dotée de cavités présentant une pluralité de points conducteurs d'émission électromagnétique distincts formés par des parties de spires d'une bobine disposés en retrait par rapport à la surface inférieure/ interne de la sonde.

Dans une autre évolution de la technique, le brevet français 2 589 067 décrit un procédé et un dispositif basés sur la constatation qu'un champ électrique appliqué à la peau en combinaison avec un rayonnement électromagnétique laser permet de modifier les propriétés d'absorption de ladite peau.

### EXPOSE DE L'INVENTION

La présente invention a pour objet une sonde pour l'application d'un flux d'énergie électromagnétique haute fréquence sur la peau (utilisable notamment dans les procédés décrits dans WO 93/04636 et brevet français 2 589 067, ou autre procédé nécessitant l'application d'un gel conducteur), cette sonde présentant une surface d'appui non-conductrice destinée à être appliquée sur la peau et présentant dans son épaisseur au moins une cavité débouchant par un orifice dans la surface d'appui ainsi qu'au moins un élément inducteur de champ électromagnétique destiné à émettre un champ électromagnétique à travers ladite cavité et son orifice.

La sonde selon l'invention est caractérisée en ce que ledit élément inducteur se trouve dans son épaisseur à un niveau intermédiaire de la profondeur de ladite cavité, cet élément inducteur s'étendant au moins partiellement autour et/ou au voisinage de la paroi de ladite cavité. Lors de l'utilisation de la sonde, on applique par exemple un mélange de gel/ produit traitant sur la surface de la peau à traiter et/ou dans les cavités de la surface d'appui de la sonde de manière que ce mélange serve d'interface conducteur entre la surface d'appui de la sonde flexible et la peau.

La sonde selon l'invention présente divers avantages. Par exemple, la disposition intermédiaire de l'élément inducteur de champ magnétique permet d'agrandir la cavité et/ou de la rendre traversante dans l'épaisseur de la sonde, de manière à pouvoir installer une source d'émission électromagnétique laser dans l'épaisseur ou sur la face supérieure/externe de la sonde. Cet arrangement permet l'application simultanée aisée d'un champ électromagnétique et d'un rayonnement électromagnétique laser sur la peau, comme décrit dans le brevet français 2 589 067. En outre, la disposition intermédiaire de l'élément inducteur de champ magnétique permet une fabrication aisée des sondes par perforation. Par ailleurs, la disposition des éléments inducteurs de champ magnétique au moins partiellement autour et/ou au voisinage de la paroi des cavités permet de les disposer en couches successives, ce qui permet de multiplier le nombre de points d'émission de champ électromagnétique dans chaque cavité. Cette disposition permet en outre d'augmenter la quantité de mélange gel/ produit traitant dans les cavités soumise au champ électromagnétique produit par les multiples points d'émission. Ceci augmente l'intensité du champ électromagnétique appliqué au mélange gel/ produit et à la peau, et ainsi le pouvoir traitant des produits appliqués.

Les éléments inducteurs de champ magnétique sont constitués soit par des spires aplaties, soit par un réseau ou une grille constituée de matériau conducteur. Le matériau conducteur est de préférence une feuille conductrice en résine de silicone, un tissu de fibres conductrices telles des fibres de carbone, ou un textile non tissé dont les fibres sont conductrices.

Dans les formes d'exécution comprenant une source de rayonnement électromagnétique laser, celle-ci est constituée soit par une diode d'émission laser logée dans l'épaisseur ou sur la face supérieure/externe de la sonde, soit par une fibre optique reliée à une source laser externe à la sonde. Dans ces mêmes formes d'exécutions, un arrangement possible est de disposer les cavités pratiquées dans la surface d'appui en réseau ou en lignes formant des figures géométriques diverses, et de disposer les sources laser de manière régulière dans ledit réseau ou lesdites lignes.

La sonde selon l'invention peut être utilisée, en combinaison avec un gel traitant, pour le traitement cosmétique de la peau, notamment l'épilation longue durée, la calvitie, la couperose et les varicosités veineuses et capillaires.

### BREVE DESCRIPTION DES DESSINS

D'autres caractéristiques de l'invention ressortiront à la lecture de la description qui suit, donnée à titre d'exemple, en se référant aux dessins schématiques annexés dans lesquels :
la Figure 1 est une vue en perspective d'une première forme d'exécution de la sonde selon l'invention, constituée d'un corps maniable allongé avec source laser.
la Figure 2 est une vue en coupe axiale selon la ligne II-II de la sonde représentée à la Figure 1.
la Figure 3 est une vue en perspective, de dessous, d'une deuxième forme d'exécution de la sonde selon l'invention.
la Figure 4 est une vue en coupe selon la ligne IV-IV de la sonde représentée à la Figure 3.
La Figure 5 est une vue en perspective, de dessous, d'une troisième forme d'exécution de la sonde selon l'invention; et
la Figure 6 est une vue en coupe selon la ligne VI-VI de la sonde représentée à la Figure 5.

### DESCRIPTION DETAILLEE

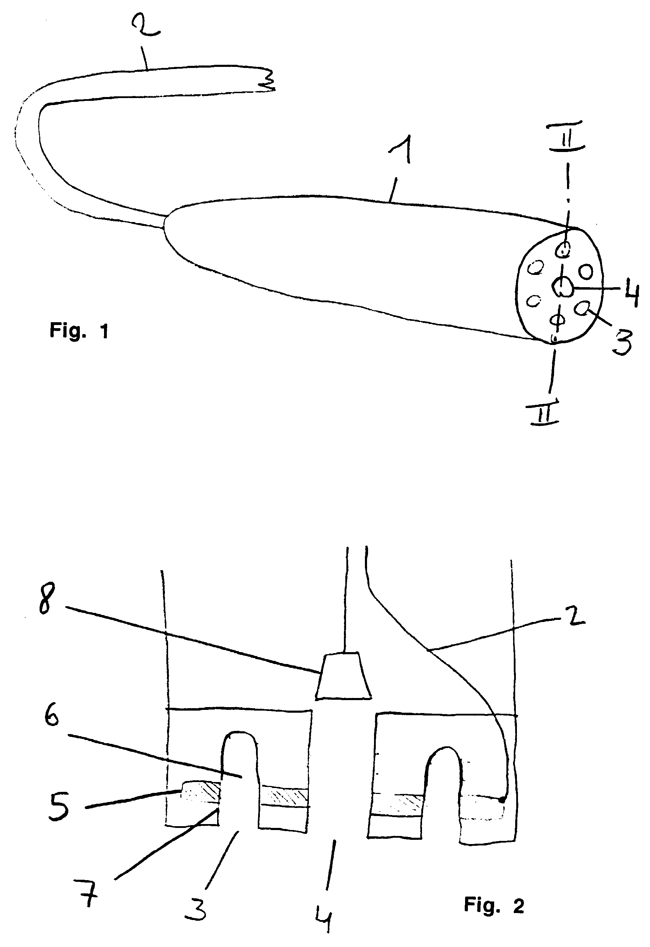

La sonde représentée aux Figures 1 et 2 comporte un organe maniable de contact 1, relié, à l'une de ses extrémités, à une amenée de courant 2 alimentant au moins un élément inducteur de champ électromagnétique 5 disposé en retrait de la surface de contact, émettant à travers des cavités 6, au nombre de six dans cet exemple. L'élément inducteur de champ électromagnétique 5 est situé à un niveau intermédiaire de la profondeur des cavités 6, cet élément inducteur s'étendant au moins partiellement autour et/ou au voisinage de la paroi 7 de ladite cavité. Une source de rayonnement électromagnétique laser 8 émet à travers une cavité additionnelle 4 située au centre des cavités 6. Les cavités 6 ont typiquement un diamètre situé entre 3mm et 6mm. La cavité 4 d'émission de rayonnement laser a typiquement un diamètre situé entre 6mm et 10mm.

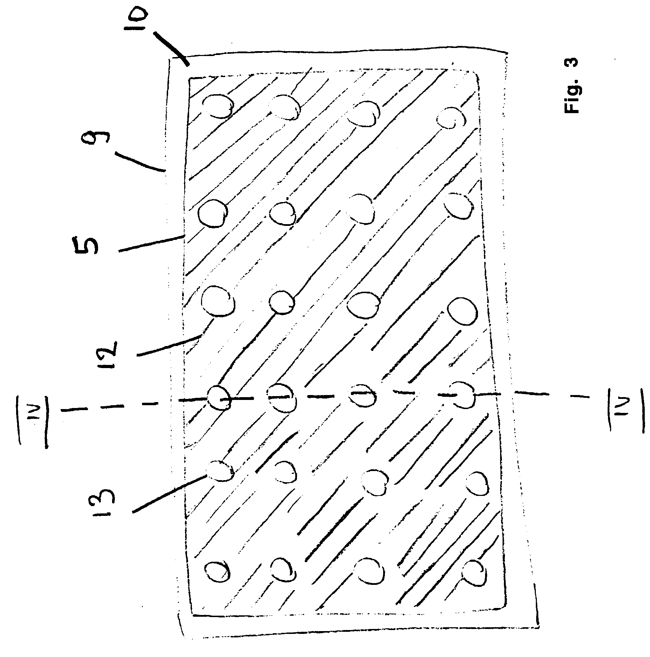

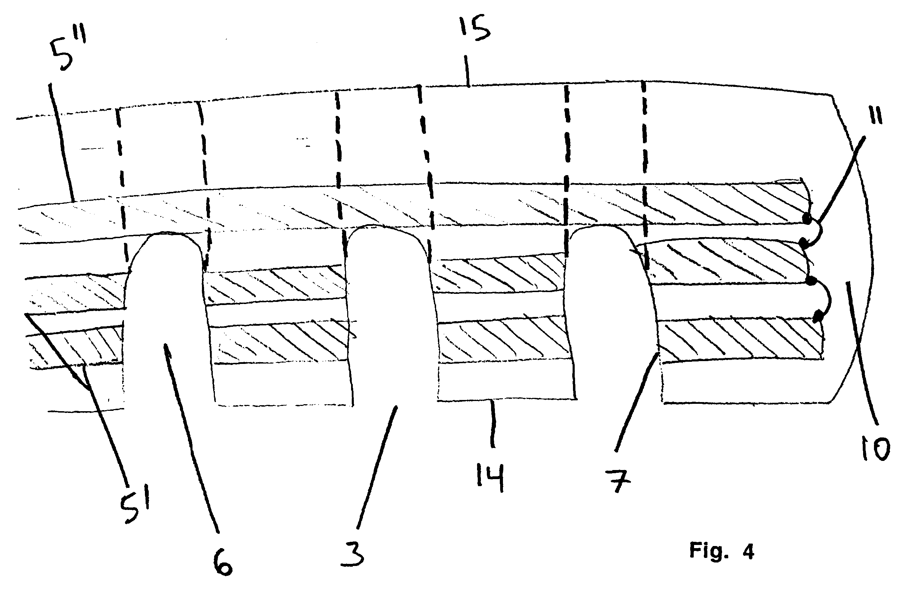

La forme d'exécution représentée aux Figures 3 et 4 est une sonde flexible 9 constituée d'une ou plusieurs feuilles de caoutchouc flexible incorporant un ou plusieurs éléments inducteurs 5 occupant la quasi-totalité de la feuille flexible, excepté son pourtour non-conducteur 10. Les éléments inducteurs 5 sont arrangés en plusieurs couches connectées électriquement entre elles par les connections 11. On distingue sur la Figure 4 des éléments inducteurs 5' situés à un niveau intermédiaire de la profondeur des cavités 6, ainsi qu un élément inducteur 5'' optionnel situé au fond des cavités 6. L'élément inducteur 5 est par exemple constitué d'un tissu de fibres de carbone tissé 12 perforé par des perforations 13. La sonde flexible présente une face inférieure/interne 14 et une face supérieure/externe 15. Les cavités 6 sont situés dans l'épaisseur de la feuille en matériau flexible et débouchent dans la surface inférieure/interne 14 par un orifice 3. En pointillé sur la Figure 4, une forme d'exécution de la sonde 9 est représentée où les cavités 6 sont perforantes et traversantes, ce qui facilite la fabrication.

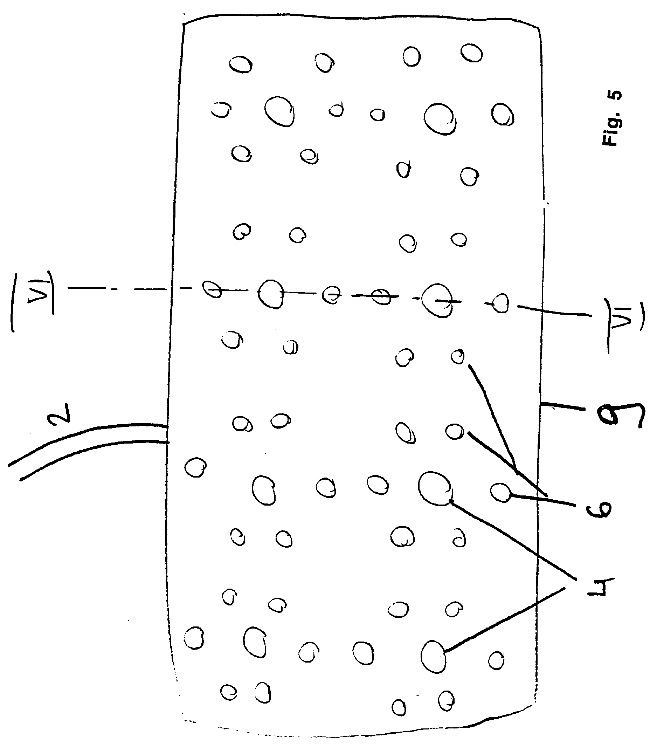

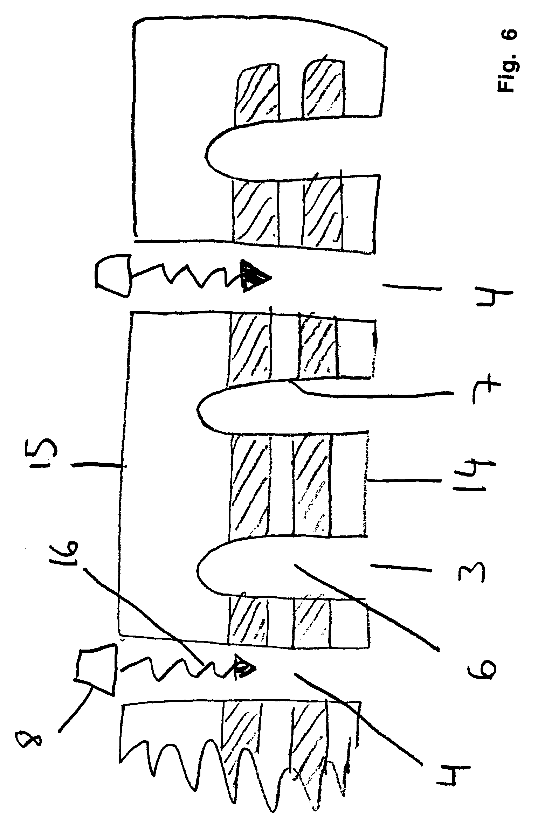

La forme d'exécution représentée aux Figures 5 et 6 est une sonde flexible 9 comportant des sources d'émission laser 8, avec une face inférieure/interne 14 et une face supérieure/externe 15. La sonde comporte d'une part des cavités borgnes 6 disposées en réseau formant des figures géométriques, par exemple selon une disposition circulaire, et d'autre part des cavités perforantes additionnelles 4 disposées de manière régulière dans ledit réseau permettant le passage du rayonnement électromagnétique laser 16. Les cavités 6 entourent des cavités perforantes 4 de manière à obtenir une répartition uniforme du rayonnement eléctromagnétique induit et du rayonnement laser. La sonde est reliée par une amenée de courant 2 à des moyens générateurs d'impulsions électriques ainsi qu'à des moyens d'excitation de sources laser. Des exemples de ces deux types de moyens sont décrits par exemple dans le brevet français 2 589 067.

Typiquement, les impulsions créant le champ électromagnétique sont des impulsions d'activation statique ayant des durées allant de 1 micros. à 1s, et ayant des fréquences de modulation à rapport constant de 5Hz à 1000Hz. Le rayonnement laser a normalement une énergie de 0.5 mW à 150 mW. Cependant, on peut utiliser des énergies plus élevées, de 500 mW et davantage, dans la mesure où le faisceau est défocalisé par une lentille afin de créer un champ de plus grande surface.

Le gel chargé utilisé pour le procédé de traitement est de préférence composé d'un gel conducteur non-polymérisable d'un type usuel pour le couplage ultrasonique d'électrodes sur la peau, en mélange avec un produit traitant. Le gel a un pH neutre et est, par exemple, à base gélifiante composée de trithanolaminepropylène glycol-carboxyvinylique. La composition du produit traitant dépend de l'action souhaitée. Pour une action dépilatoire, on peut choisir un produit à effet d'atrophie progressif de la racine du poil, par exemple une lotion post-épilatoire du type utilisé d'habitude immédiatement après une épilation à la cire, ainsi que les jours suivants. De telles lotions comportent des extraits de plantes, des huiles essentielles, de l'eau déminéralisée et éventuellement d'autres composants, par exemple des polyoxyéthylènes. Ces produits, connus parfois sous la dénomination "modérateur de la repousse des poils", sont sans danger d'utilisation toxique ou autre.

Pour un traitement de la calvitie, on peut mélanger le gel avec, par exemple, du minoxydil, ou tout autre produit favorisant la repousse des cheveux. Un mélange de 50:50 vol% de gel et de minoxydil a donné des résultats satisfaisants.

Afin de ne pas compromettre les propriétés conductrices du gel, la part du produit actif ou traitant n'excédera en général pas 50%, usuellement moins de 25%, en poids du gel (% en poids = % en volume). On peut ajouter de l'alcool, du chlorure de sodium et/ou d'autres substances pour améliorer la conductivité du produit, et/ou comme agents conservateurs.

Des tests ont démontré que l'application d'une énergie à haute fréquence sur le produit traitant seul ou sur le gel seul n'aboutit à aucun effet spécial, tandis qu'en mélange, on obtient une bonne pénétration du produit traitant entraîné par la solution conductrice provenant du gel. Il semble que la conduction de l'énergie électromagnétique et électrique suit le chemin de moindre résistance offert par le mélange du gel et de la lotion conductrice appliqué à la surface de la peau, et ensuite autour de la racine du poil ou du cheveu par la seule lotion conductrice qui pénètre dans le follicule pileux et la tige du poil. Le gel permet un dégagement progressif de la lotion active et sa pénétration, sous l'action conjuguée des champs électromagnétique et électrique. Dans les formes d'exécution comportant des couches successives d'éléments inducteurs, la multiplication des points d'émission de champ électromagnétique et la quantité plus importante de mélange gel/produit traitant présent dans les cavités permet une pénétration plus complète du produit traitant. Cet effet est en outre renforcé par l'adjonction d'un rayonement électromagnétique laser.

## Revendications

1. Sonde pour l'application d'un flux d'énergie électromagnétique haute fréquence sur la peau, la sonde présentant une surface d'appui non-conductrice (14) destinée à être appliquée sur la peau et présentant dans son épaisseur au moins une cavité (6) débouchant par un orifice (3) dans la surface d'appui ainsi qu'au moins un élément inducteur de champ électromagnétique (5) destiné à émettre un champ électromagnétique à travers ladite cavité et son orifice (3),
caractérisée en ce que ledit élément inducteur (5) se trouve dans ladite épaisseur à un niveau intermédiaire de la profondeur de ladite cavité (6), cet élément inducteur s'étendant au moins partiellement autour et/ou au voisinage de la paroi de ladite cavité.

2. Sonde selon la revendication 1, caractérisée en ce que le ou les éléments inducteurs sont constitués de spires de forme aplatie.

3. Sonde selon la revendication 1, caractérisée en ce que le ou les éléments inducteurs sont constitués d'un matériau conducteur plan dans lequel des perforations ou découpes ont été pratiquées.

4. Sonde selon la revendication 1, caractérisée en ce que le ou les éléments inducteurs sont constitués d'une trame tissée, chaque brin de la trame étant constituée d'un matériau conducteur.

5. Sonde selon la revendication 1, caractérisée en ce que le ou les éléments inducteurs sont constitués d'un textile non tissé dont les fibres sont en matériau conducteur.

6. Sonde selon la revendication 1, caractérisée en ce que le ou les éléments inducteurs sont constitués d'une trame tissée en fibre de carbone.

7. Sonde selon la revendication 1, caractérisée en ce que au moins deux éléments inducteurs de champ magnétique sont disposés en deux ou plusieurs couches distinctes.

8. Sonde selon la revendication 7, caractérisée en ce qu'elle comporte en outre un élément inducteur additionnel (5'') situé au fond de la ou des cavités pratiquées dans la surface d'appui.

9. Sonde selon l'une quelconque des revendications 1 à 8, caractérisée en ce qu'elle comporte au moins une source de rayonnement électromagnétique laser (8) disposée dans au moins une cavité ou trou traversant (4) pratiqué dans la surface d'appui, de manière à agir de concert avec le rayonnement électromagnétique.

10. Sonde selon la revendication 9, caractérisée en ce que la source laser est constituée par au moins une diode d'émission laser intégrée à la sonde ou au moins une fibre optique reliée à au moins un dispositif d'émission laser externe à la sonde.

11. Sonde selon l'une quelconque des revendications 1 à 10, caractérisée en ce qu'elle comporte un organe maniable de contact avec la peau (1), cet organe étant constitué d'un corps allongé dont l'une des faces forme la surface d'appui.

12. Sonde selon l'une quelconque des revendications 1 à 10, caractérisée en ce que qu'elle se présente sous forme d'une feuille flexible susceptible de se conformer à la partie du corps contre laquelle elle est appliquée, cette feuille flexible comportant des parties inférieure/interne (14) et supérieure/externe (15) non-conductrices, au moins ladite surface inférieure comportant ladite surface d'appui.

13. Sonde selon l'une quelconque des revendications 9 à 12, caractérisée en ce que les cavités pratiquées dans la surface d'appui sont disposées en réseau ou en lignes formant des figures géométriques diverses, en ce que les sources laser sont disposées de manière régulière dans ledit réseau ou lesdites lignes.

14. Sonde selon l'une quelconque des revendications 7 à 13, caractérisée en ce qu'elle est formée de plusieurs feuilles distinctes empilées en sandwich, en ce que les cavités ou trous traversants sont produits par perforation.

15. Utilisation de la sonde selon l'une quelconque des revendications 1 à 14 en combinaison avec un mélange de gel conducteur et d'un produit traitant pour le traitement cosmétique de la peau.

16. Utilisation de la sonde selon l'une quelconque des revendications 1 à 14 en combinaison avec un mélange de gel conducteur et d'un produit à effet d'atrophie sur la racine des poils pour une épilation de longue durée.

17. Utilisation de la sonde selon l'une quelconque des revendications 1 à 14 en combinaison avec un mélange de gel conducteur et d'un produit régénérateur des cheveux pour un traitement cosmétique de la calvitie.

18. Procédé de traitement cosmétique de la peau, notamment utilisant la sonde selon l'une quelconque des revendications 1 à 14, suivant lequel on applique sur la peau un mélange de gel conducteur et d'un produit traitant, et on émet dans ce mélange un courant électromagnétique de haute fréquence pour amener le produit à pénétrer dans les pores de la peau, caractérisé
en ce que l'on applique aussi, simultanément ou non, un rayonnement électromagnétique laser sur la peau afin d'augmenter l'effet, la durée d'action et la pénétration du produit traitant.

19. Procédé selon la revendication 18 pour une épilation de longue durée, caractérisé en ce qu'on applique sur la peau un mélange de gel conducteur et d'un produit à effet d'atrophie sur la racine des poils.

20. Procédé selon la revendication 18 pour un traitement cosmétique de la calvitie, caractérisé en ce que l'on applique sur la peau un mélange de gel conducteur et d'un produit régénérateur des cheveux.

21. Procédé selon la revendication 18 pour un traitement cosmétique de la couperose, caractérisé en ce que l'on applique sur la peau un mélange de gel conducteur et d'un produit a effet d'atrophie sur les capillaires sanguins.

22. Procédé selon la revendication 18 pour un traitement cosmétique des varicosités veineuses et capillaires, caractérisé en ce que l'on applique sur la peau un mélange de gel conducteur et d'un produit a effet d'atrophie sur les veines et les capillaires sanguins.
